# EUROPÄISCHE PATENTSCHRIFT

(11) **EP 1 435 994 B1**
(45) Veröffentlichungstag und Bekanntmachung des Hinweises auf die Patenterteilung: **19.05.2010**
(21) Anmeldenummer: 02777301.9
(22) Anmeldetag: 10.10.2002
(51) Int. Cl.: A61K 38/17, A61P 11/00

(54) **NEUE VERWENDUNG VON LUNGENSURFACTANT**
NOVEL USE OF PULMONARY SURFACTANT
NOUVELLE UTILISATION DE SURFACTANT PULMONAIRE

(30) Priorität: 11.10.2001 EP 01000531
(43) Veröffentlichungstag der Anmeldung: 14.07.2004
(73) Patentinhaber: Nycomed GmbH, 78467 Konstanz (DE)
(72) Erfinder: TAUT, Friedemann, 78464 Konstanz (DE)
(74) Vertreter: Kratzer, Bernd
(86) Internationale Anmeldenummer: PCT/EP2002/011325
(87) Internationale Veröffentlichungsnummer: WO 2003/033014

(56) Entgegenhaltungen:
- WO-A-01/58423
- HÄFNER D ET AL: "Additive effects of phosphodiesterase-4 inhibition on effects of rSP-C surfactant." AMERICAN JOURNAL OF RESPIRATORY AND CRITICAL CARE MEDICINE. UNITED STATES MAY 2000, Bd. 161, Nr. 5, Mai 2000 (2000-05), Seiten 1495-1500, XP002227662 ISSN: 1073-449X
- TOKIEDA K ET AL: "Surfactant protein-B-deficient mice are susceptible to hyperoxic lung injury." AMERICAN JOURNAL OF RESPIRATORY CELL AND MOLECULAR BIOLOGY. UNITED STATES OCT 1999, Bd. 21, Nr. 4, Oktober 1999 (1999-10), Seiten 463-472, XP002227663 ISSN: 1044-1549
- GHIO A J ET AL: "SYNTHETIC SURFACTANT SCAVENGES OXIDANTS AND PROTECTS AGAINST HYPEROXIC LUNG INJURY" JOURNAL OF APPLIED PHYSIOLOGY: RESPIRATORY, ENVIRONMENTAL AND EXERCISE PHYSIOLOGY, AMERICAN PHYSIOLOGICAL SOCIETY, US, Bd. 77, Nr. 3, 1. September 1994 (1994-09-01), Seiten 1217-1223, XP000602470 ISSN: 0161-7567
- HUANG Y-C ET AL.: "Artificialsurfactant attenuates hyperoxic lung injury in primates, I" J. APPLIED PHYSIOLOGY, Bd. 78, Nr. 5, 1995, Seiten 1816-1822, XP009087026
- WILSON D. ET AL: "effect of exogenous surfactant (Calfactant) in pediatric acute lung injury" JAMA, Bd. 293, Nr. 4, 26. Januar 2005 (2005-01-26), Seiten 470-476,

## Beschreibung

### Technisches Gebiet der Erfindung

Die Erfindung betrifft die neue Verwendung von Lungensurfactant-Zubereitungen bei der künstlichen Beatmung mit toxischen Sauerstoffkonzentrationen von Patienten mit akutem Atemnotsyndrom (ARDS) aufgrund von Lungenentzündung oder Aspiration von Mageninhalt.

### Stand der Technik

In der Beatmungstherapie von Patienten mit Lungenversagen ist es ein vordringliches Ziel, die inspiratorische, also am Beatmungsgerät eingestellte inspiratorische Sauerstoffkonzentration (FiO2, fraction of inspired oxygen) möglichst niedrig zu halten. Die mit einer erhöhten (ca. > 0,5) Fi02 einhergehenden Risiken sind u.a.:
- Entstehung von höheren Konzentrationen an freien Sauerstoffradikalen O*, die Protein- oder Lipidstrukturen der Lunge oxidieren und somit in ihrer Funktion beeinträchtigen können.
- Schädigung des Lungensurfactant durch Hyperoxie, wobei unter anderem ebenfalls oxidative Prozesse an der Zerstörung von Surfactanthestandtellen (z. B. Lipidperoxidation) beteiligt sind.
- Initiierung oder Verstärkung der Entzündungskaskade mit Mediatorenfreisetzung und Immunzellaktivierung in Lungengewebe.
- In minderbelüfteten Lungenarealen besteht die Gefahr der Resorptionsatelektasen. Wenn das inspiratorische Gasgemisch zu großen Teilern aus Sauerstoff besteht, kann dieser ins Blut aufgenommen (resorbiert) werden, so dass der Gasinhalt einer betroffenen Alveole stark abnimmt. Es kommt zur Atelektase, also zum Kollaps der Alveole mit der Folge einer Erhöhung des intrapulmonalen Shunts. (Durchblutete, aber nicht belüftete Alveolen lassen anteilig venöses Blut in den arteriellen Kreislauf gelangen, wodurch der Sauerstoffgehalt des arteriellen Blutes stark abnehmen kann).
- Toxisch hohe Sauerstoffkonzentrationen können per se ein ARDS (Adult Respiratory Distress syndrome bzw. Acute Respiratory Distress Syndrome) auslösen.
- Nachteilige feingewebliche Veränderungen in der Lunge sind schon nach 24 bis 48 Stunden hyperoxischer Beatmung feststellbar.

In US 4765987 wird in Beispiel 5 die Untersuchung der Effizienz und klinischen Sicherheit von artifiziellem Surfactant bei der Behandlung von Frühgeborenen untersucht. Dem Fachmann ist bekannt, dass Frühgeborene selbst noch kein Surfactant bilden können. Die Untersuchung wird nicht mit Menschen durchgeführt, die Surfactant selbst bilden können und mit toxischen Sauerstoffkonzentration beatmet werden müssen.

In Shamolov et al. (Shamolov VY et al. (1999) ROSSIISKII VESTNIK PERINATOLOGII I PEDIATRII, Bd. 44. Nr. 4, Seiten 29-34) wird die Effektivität von natürlichem Surfactant erhalten aus Fruchtwasser an Neugeborenen untersucht.

Zur Toxizität von erhöhten Sauerstoffkonzentrationen in der Einatemluft sei weiterhin auf die Übersichtsarbeit von Lodato verwiesen (Lodato, R.F., Critical Care Clinics, 6:749-765)

Der Arzt ist daher stets bestrebt, eine ausreichende Oxigenierung des künstlich beatmeten Patienten mit der geringst möglichen inspiratorischen Sauerstoffkonzentration zu erzielen. Trotz ausgefeiltester Beatmungstechniken (Einsatz von PEEP, sonstige Rekrutierungsmaßnahmen, Optimierung der Beatmungsdrücke, Veränderung des Atemzeitverhältnisses) ist es jedoch bei Patienten mit schwerem Lungenversagen oft unumgänglich, die Fi02 über 0,5 bis hin zu 1,0 einzustellen.

Aufgabe der vorliegenden Erfindung ist es daher ein Arzneimittel bereitzustellen, das den Patienten vor den Risiken und Nebenwirkungen bei der Beatmung mit toxischen Sauerstoffkonzentration schützt.

### Beschreibung der Erfindung

Überraschenderweise wurde nun gefunden, dass nach Verabreichung von Lungensurfactant Zubereitungen an Patienten, die künstlich mit toxischen Sauerstoffkonzentrationen beatmet werden, im Vergleich zu beatmeten Patienten denen keine Lungensurfactant Zubereitung verabreicht wurde, die Konzentration des Sauerstoffes im inspiratorischen Beatmungsgas wesentlich schneller abgesenkt werden konnte auf weniger toxische oder nicht mehr toxische Konzentrationen unter Beibehaltung einer adäquaten Oxigenierung des arteriellen Blutes. Die mit der Beatmung mit toxischen Sauerstoffkonzentrationen verbundenen Risiken werden vermindert und die Mortalität gesenkt. Weiterhin ist zu erwarten, dass die Beatmungstherapie früher beendet werden kann. Der Aufenthalt von Patienten auf Intensivstationen kann verkürzt werden und somit Kosten eingespart werden.

Gegenstand der Erfindung ist daher die Verwendung einer Lungensurfactant-Zubereitung zur Herstellung von Arzneimitteln zur Prophylaxe oder Behandlung von Patienten mit akutem Atemwegsyndrom (ARDS) aufgrund von Lungenentzündung oder Aspiration von Mageninhalt.

Unter toxischen Sauerstoffkonzentration werden erfindungsgemäß inspiratorische Sauerstoffkonzentration des zur Beatmung eingesetzten Gases verstanden bei denen der Sauerstoffanteil 50 Vol. % oder mehr beträgt (FiO2 ≥ 0.5). Bevorzugt werden unter toxischen Sauerstoffkonzentration inspiratorische Sauerstoffkonzentration des zur Beatmung eingesetzten Gases verstanden bei denen der Sauerstoffanteil 75 Vol. % oder mehr beträgt (FiO2 ≥ 0.75).

Erfindungsgemäß wird unter künstlicher Beatmung eines Patienten die durch Hilfsmittel bewirkte Belüftung (Ventilation) der Lunge verstanden. Als beispielhaftes Hilfsmittel für die Beatmung sei der Respirator genannt, wobei unterschiedliche, dem Fachmann bekannte Beatmungsformen zur Anwendung kommen können.

Bei Patienten mit schwerem Lungenversagen, die beatmet werden, handelt es sich insbesondere um Patienten mit schwerer Oxigenierungsstörung, die einen Quotienten aus arteriellem SauerstoffPartialdruck (Pa02) und inspiratorischer Sauerstoffkonzentration (FiO2) von 200 mm Hg oder niedriger haben (PaO2/FiO2 ≤ 200 mm Hg).

Erfindungsgemäß bevorzugt handelt es sich bei den Patienten um Menschen, vorzugsweise nicht um Frühgeborene oder Neugeborene mit IRDS (Infant Respiratory Distress Syndrome).

Natürlicher Lungensurfactant hat oberflächenaktive Eigenschaften; er reduziert beispielsweise die Obemächenspannung in den Lungenbläschen. Einen einfachen und schnellen in vitro Test mit dem sich die Oberflächenaktivität von Lungensurfactant bestimmen lässt, stellt z.B. die sogenannte Wilhelmy Waage dar [Goerke, J. Biochim. Biophys. Acta, 344: 241-261 (1974), King R.J. and Clements J.A., Am. J. Physicol. 223: 715-726 (1972)]. Diese Methode gibt Hinweise auf die Lungensurfactant-Qualität, gemessen als Tätigkeit eines Lungensurfactants, eine Oberflächenspannung von nahe Null mN/m zu erreichen. Eine andere Messvorrichtung, um die Oberflächenaktivität von Lungensurfactant zu bestimmen, ist der "Pulsating Bubble Surfactometer" [Possmayer F., Yu S. und Weber M., Prog. Resp. Res., Ed. v. Wichert, Vol. 18: 112-120 (1984)].

Die Aktivität einer Lungensurfactant-Zubereitung kann auch mittels in vivo Tests festgestellt werden, beispielsweise so, wie von Häfner et al. beschreiben (D. Häfner et al.: Effects of rSP-C surfactant on oxygenation and histology in a rat lung lavage model of acute lung injury. Am. J. Respir. Crit. Care Med. 1998, 158: 270-278). Durch die Messung von z.B. der Lungencompliance, des Blutgasaustausches bzw. der benötigten Beatmungsdrücke kann man Hinweise auf die Aktivität eines Lungensurfactants erhalten.

Unter Lungensurfactant-Zubereitung werden erfindungsgemäß die zahlreichen bekannten Zusammensetzungen und deren Abwandlungen verstanden, die die Funktion von natürlichem Lungensurfactant besitzen. Bevorzugt sind dabei die Zusammensetzungen, die beispielsweise in den oben beschriebenen Tests Aktivität aufweisen. Besonders bevorzugt sind diejenigen Zusammensetzungen, die in einem solchen Test im Vergleich zu natürlichem, insbesondere humanen Lungensurfactant, eine erhöhte Aktivität zeigen. Es kann sich hierbei um Zusammensetzungen handeln, die nur Phospholipide enthalten, aber auch um Zusammensetzungen, die außer den Phospholipiden unter anderem noch Lungensurfactant-Protein enthalten. Bevorzugte erfindungsgemäße Phospholipide sind Dipalmitoylphosphatidylcholin (DPPC), Palmitoyloleylphosphatidylglycerol (POPG) und/oder Phosphatidylglycerol (PG). Besonders bevorzugt handelt es sich bei den Phospholipiden um Mischungen aus verschiedenen Phospholipiden, insbesondere um Mischungen aus Dipalmitoylphosphatidylcholin (DPPC) und Palmitoyloleylphosphatidylglycerol (POPG), bevorzugt im Verhältnis von 7 zu 3 bis 3 zu 7. An Handelsprodukten sind zu nennen Curosurf® (Serono, Pharma GmbH, Unterschleißheim), ein natürliches Surfactant aus homogenisierten Schweinelungen, Survanta® (Abbott GmbH, Wiesbaden) und Alveofact® (Boehringer Ingelheim), beides Extrakte aus Rinderlungen, sowie Exosurf® (Glaxo Wellcome), ein synthetisches Phospholipid mit Hilfsstoffen. Als Lungensurfactant-Proteine kommen sowohl die aus natürlichen Quellen, wie beispielsweise Lungen-Lavage oder Extraktion aus Fruchtwasser, gewonnenen als auch die gentechnisch bzw. chemisch synthetisch hergestellten Proteine in Frage. Erfindungsgemäß sind insbesondere die mit SP-B und SP-C bezeichneten Lungensurfactant-Proteine und deren modifizierte Derivate von Interesse. Die Aminosäuresequenzen dieser Lungensurfactant-Proteine, ihre Isolierung bzw. gentechnologische Herstellung sind bekannt (z.B. aus WO86/03408. EP-A-0 251 449, WO89/04326, WO87/06943, WO88/03170, WO91/00871, EP-A-O 368 823 und EP-A-O 348 967). Modifizierte Derivate der mit SP-C bezeichneten Lungensurfactant-Proteine, die sich vom humanen SP-C durch den Austausch einiger Aminosäuren unterscheiden, sind z.B. in WO91/18015 und WO95/32992 beschrieben. Besonders hervorzuheben sind in diesem Zusammenhang die rekombinanten SP-C Derivate, die in WO95/32992 offenbart sind, insbesondere diejenigen, die sich von humanem SP-C in den Positionen 4 und 5 durch den Austausch von Cystein gegen Phenylalanin und in der Position 32 durch den Austausch von Methionin gegen Isoleucin unterscheiden [im folgenden als rSP-C (FF/I) oder Lusupultid (INN) bezeichnet]. Unter modifizierten Derivaten der Lungensurfactant-Proteine sollen auch solche Proteine verstanden werden, die eine vollkommen eigenständig im Hinblick auf ihre Lungensurfactant-Eigenschaft konzipierte Aminosäuresequenz aufweisen, wie sie beispielsweise in EP-A-O 593 094 und WO 92/22315 beschrieben sind. Bevorzugt genannt sei in diesem Zusammenhang das Polypeptid KL4 (INN: Sinapultid). Die Bezeichnung Lungensurfactant-Protein umfasst erfindungsgemäß auch Mischungen unterschiedlicher Lungensurfactant-Proteine. In EP-B-0 100 910, EP-A-0 110 498, EP-B-0 119 056, EP-B-0 145 005 und EP-B-0 286 011 sind Phospholipid-Zusammensetzungen mit und ohne Lungensurfactant-Proteine beschrieben, die ebenfalls als Komponenten der Zubereitungen in Frage kommen.

Als weitere Bestandteile die in Lungensurfactant-Zubereitungen vorhanden sein können, seien Fettsäuren wie Palmitinsäure genannt Die Lungensurfactant-Zubereitungen können auch Elektrolyte wie Calcium-, Magnesium- und/oder Natriumsalze (beispielsweise Calciumchlorid, Natriumchlorid und/oder Natriumhydrogencarbonat) enthalten, um eine vorteilhafte Viskosität einzustellen. Bevorzugte erfindungsgemäße Zubereitungen enthalten 80 bis 95 Gew.-% Phospholipide, 0,5 bis 3,0 Gew.-% Lungensurfactant-Proteine, 3 bis 15 Gew.-% Fettsäure, vorzugsweise Palmitinsäure, und 0 bis 3 Gew.-% Calciumchlorid.

Die Lungensurfactant-Zubereitungen werden nach an sich bekannten, dem Fachmann geläufigen Verfahren hergestellt, beispielsweise wie in der WO95/32992 beschrieben. Erfindungsgemäß bevorzugt handelt es sich bei den Lungensurfactant-Zubereitungen um lyophilisierte und insbesondere um sprühgetrocknete Lungensurfactant-Zubereitungen. Lyophilisierte Zubereitungen sind beispielsweise bekannt aus WO97/35882, WO91/00871 und DE 3229179. Die WO97/26863 beschreibt ein Verfahren zur Herstellung von pulverförmigen Lungensurfactant-Zubereitungen durch Sprühtrocknung. Erfindungsgemäß sind auf diese Weise hergestellte Zubereitungen bevorzugt.

Ein weiterer Gegenstand der Erfindung ist auch ein therapeutisches Verfahren zur Prophylaxe vor oder Behandlung von Risiken und Nebenwirkungen bei der künstlichen Beatmung von Patienten mit schwerem Lungenversagen durch toxischen Sauerstoffkonzentrationen. Das Verfahren ist **dadurch gekennzeichnet, dass** man dem betroffenen Patienten eine therapeutisch wirksame und pharmakologisch verträgliche Menge einer Lungensurfactant-Zubereitung verabreicht. Die Dosierung der Lungensurfactant-Zubereitungen erfolgt in der für Lungensurfactant-Zubereitungen üblichen Größenordnung.

Die Verabreichung der Lungensurfactant-Zubereitung erfolgt auf eine dem Fachmann bekannte Weise, vorzugsweise durch intratracheale Instillation (Infusion oder Bolus) einer Lungensurfactant-Lösung bzw. -Suspension oder in Form einer Vernebelung einer Lungensurfactant-Lösung bzw. -Suspension oder durch Vernebelung von Lungensurfactant-Pulver. Bevorzugt werden die erfindungsgemäßen Zubereitungen für die Verabreichung in einem geeigneten Lösungsmittel oder Resuspendiermedium gelöst bzw. suspendiert, insbesondere wenn die Zubereitungen in lyophilisierter oder sprühgetrockneter Form vorliegen. Vorzugsweise handelt es sich bei dem geeigneten Resuspendiermedium um physiologische Kochsalzlösung. Es erweist sich als vorteilhaft, Suspensionen bzw. Lösungen der erfindungsgemäßen Zubereitungen zu verabreichen, die 12,5 bis 100 mg Phospholipide pro ml Suspension enthalten. Vorzugsweise werden die erfindungsgemäßen Zubereitungen pro Applikation in einer solchen Menge verabreicht, dass die Menge an Phospholipiden zwischen 12,5 und 200 mg pro Kilogramm Körpergewicht beträgt. Die Verabreichung erfolgt in der Regel ein- bis dreimal täglich über einen Zeitraum von 1 bis 7 Tagen. Bevorzugt ist ein Verfahren bei dem die eingesetzte Lungensurfactant Lösung 0,5 bis 2,0 mg rSP-C (FF/I) pro ml Lösungsmittel enthält. Besonders erwähnt sei ein Verfahren bei dem die eingesetzte Lungensurfactant-Lösung 0,75 bis 1,5 mg rSP-C (FF/I) pro ml Lösungsmittel enthält. Gewünschtenfalls kann vor der Verabreichung der erfindungsgemäßen Zubereitungen eine bronchoalveoläre Lavage, vorzugsweise mit verdünnter Lungensurfactant-Zubereitung durchgeführt werden. Eine solche Vorgehensweise ist beispielsweise beschrieben in Gommers et al. [Bronchoalveolar lavage with a diluted surfactant suspension prior to surfactant instillation improves the effectiveness of surfactant therapy in experimental acute respiratory distress syndrome (ARDS), Intensive Care Med. 1998, 24:494-500] und in der WO98/49191.

Ein weiterer Gegenstand der Erfindung ist ein Handelsprodukt, bestehend aus einem üblichen Sekundärpackmittel, einem eine pharmazeutische Zubereitung enthaltenden Primärpackmittel (beispielsweise eine Ampulle) und gewünschtenfalls einem Beipackzettel, wobei die pharmazeutische Zubereitung geeignet ist zur Prophylaxe oder Behandlung von Risiken und Nebenwirkungen bei der Beatmung von Patienten mit toxischen Sauerstoffkonzentration und wobei auf dem Sekundärpackmittel oder auf dem Beipackzettel des Handelsprodukts auf die Eignung der pharmazeutischen Zubereitung zur Prophylaxe oder Behandlung von Risiken und Nebenwirkungen bei der Beatmung mit toxischen Sauerstoffkonzentrationen hingewiesen wird, und wobei es sich bei der pharmazeutischen Zubereitung um eine Lungensurfactant-Zubereitung handelt. Das Sekundärpackmittel, das die pharmazeutische Zubereitung enthaltende Primärpackmittel und der Beipackzettel entsprechen ansonsten dem, was der Fachmann als Standard für pharmazeutische Zubereitungen dieser Art ansehen würde. Geeignete Primärpackmittel sind beispielsweise Ampullen oder Flaschen aus geeigneten Materialien wie durchsichtiges Polyethylen oder Glas oder auch geeignete Applikationsmittel wie sie üblicherweise zur Applikation von Wirkstoffen in die Lunge eingesetzt werden. Beispielhaft genannt seien Applikationsmittel zur Vernebelung einer WirkstoffLösung bzw. -Suspension oder zur Vernebelung von Wirkstoff-Pulver. Bevorzugt handelt es sich bei dem Primärpackmittel um eine Glasflasche, die beispielsweise durch einen handelsüblichen Gummistopfen oder ein Septum verschlossen sein kann. Als geeignetes Sekundärpackmittel sei beispielhaft eine Faltschachtel genannt.

### Beispiele

### A.) Herstellung pulverförmiger Lungensurfactant-Zubereitungen

Die Herstellung pulverförmiger Lungensurfactant-Zubereitungen erfolgt nach dem in der WO97/26863 beschriebenen Verfahren:

### Beispiel 1

7,0 g 1,2-Dipalmitoyl-3-sn-phosphatidylcholin, 2,5 g 1-Palmitoyl-2-oleoyl-3-sn-phosphatidylglycerolnatrium, 205 mg Calciumchloriddihydrat und 250 mg Palmitinsäure werden unter Erwärmen auf 60°C in 300 ml Ethanol/Wasser (85:15) gelöst, auf Raumtemperatur abgekühlt und mit 350 ml einer Lösung von rSP-C (FF/I) in Chloroform/Methanol 9:1 (c = 429 mg/l) gemischt. Die resultierende Lösung wird in einem Laborsprühtrockner Büchi B 191 sprühgetrocknet. Sprühbedingungen: Trocknungsgas Luft, Eintrittstemperatur 90°C, Austrittstemperatur 52 - 54°C. Man erhält ein lockeres Pulver.

### Beispiel 2

Eine Lösung von aus Rinderlungen gewonnenem Surfactant (erhalten durch Extraktion und Reinigungsschritte, wie z. B. beschrieben in EP 406732) in Chloroform/Methanol wird unter folgenden Bedingungen sprühgetrocknet: Laborsprühtrockner Büchi B 191, Trocknungsgas Stickstoff, Eintrittstemperatur 80°C, Austrittstemperatur 50 - 52°C. Man erhält ein feines, gelbliches Pulver.

### Beispiel 3

10,95 g 1,2-Dipalmitoyl-3-sn-phosphatidylcholin, 4,6 g 1-Palmitoyl-2-oleoyl-3-sn-phosphatidylglycerolammonium, 418 mg Calciumchloriddihydrat und 750 mg Palmitinsäure werden in 330 ml 2-Propanol/Wasser (85:15) bei 50°C gelöst und nach dem Abkühlen auf 30°C mit 620 ml einer Lösung von, rSP-C (FF/I) in Isopropanol/Wasser (95: 5, c = 484 mg/l) gemischt. Die resultierende Lösung wird in einem Laborsprühtrockner Büchi B 191 sprühgetrocknet. Sprühbedingungen: Trocknungsgas Stickstoff, Eintrittstemperatur 100°C, Austrittstemperatur 58 - 60°C. Man erhält ein farbloses Pulver.

### Beispiel 4

3,74 g (5,1 mmol) 1,2-Dipalmitoyl-3-sn-phosphatidylcholin, 2,81 g (3,7 mmol) 1-Palmitoyl-2-oleoyl-3-sn-phosphatidylcholin, 2,90 g (3,9 mmol) 1,2-Dipalmitoylphosphatidyl-3-sn-phosphatidylglycerolnatrium, 234 mg Palmitinsäure und 279 mg (1,9 mmol) Calciumchloriddihydrat werden in 160 ml 2-Propanol/Wasser (85 : 15) bei 50°C gelöst und nach dem Abkühlen auf 30°C mit 566 ml einer Lösung von rSP-C (FF/I) in Isopropanol/Wasser (92 : 8, c = 330 mg/l) bei 30°C gemischt. Die resultierende Lösung wird in einem Laborsprühtrockner Büchi B 191 sprühgetrocknet. Sprühbedingungen: Trocknungsgas Stickstoff, Eintrittstemperatur 90°C, Austrittstemperatur 58 - 60°C. Man erhält ein farbloses Pulver.

### Beispiel 5

0,5 g KL4 (INN: Sinapultid), 7,125 g 1,2-Dipalmitoyl-3-sn-phosphatidylcholin und 2,43 g 1-Palmitoyl-2-oleoyl-3-sn-phosphatidylglycerol-ammonium werden in 500 ml Chloroform/Methanol 1 : 1 unter Erwärmen auf 45°C gelöst und anschließend in einem Laborsprühtrockner Büchi B 191 sprühgetrocknet. Sprühbedingungen: Trocknungsgas Stickstoff, Eintrittstemperatur 85°C, Austrittstemperatur 55°C. Man erhält ein farbloses Pulver.

### Beispiel 6

Eine nach Beispiel 1, 3 oder 4 erhältliche Lösung von Phospholipiden, Palmitinsäure und Calciumchloriddihydrat wird - ohne Zugabe einer Lösung von rSP-C (FF/I) - entsprechend den Bedingungen nach Beispiel 1, 3 oder 4 sprühgetrocknet. Man erhält ein Pulver.

### B.) Herrichtung der erfindungsgemäßen Arzneimittel

### Beispiel 7

0.1 bis 10 g des nach Beispiel 1 erhaltenen Pulvers werden in eine Flasche mit 100 bis 250 ml Volumen abgefüllt und die Flasche wird verschlossen. Die Flasche wird zusammen mit einem Beipackzettel in eine geeignete Faltschachtel verpackt.

### Beispiel 8

3g des nach Beispiel 1 erhaltenen Pulvers werden in eine Flasche mit 66 ml Volumen abgefüllt und die Flasche wird verschlossen. Die Flasche wird zusammen mit einem Beipackzettel in eine geeignete Faltschachtel verpackt.

### Pharmakologische Untersuchungen

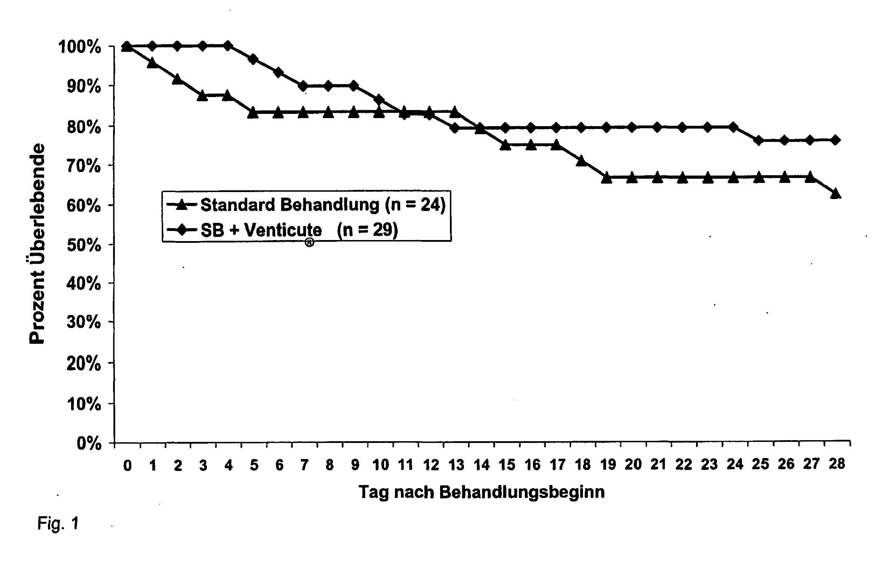

Im Rahmen von zwei prospektiven, multizentrischen, randomisierten, doppelblinden Studien mit Behandlungs- und Kontrollgruppen bei Patienten mit ARDS zeigte sich überraschenderweise, dass insbesondere diejenigen Patienten von einer Behandlung mit einer Lungensurfactant-Zubereitung profitieren, die mit toxischen Sauerstoffkonzentrationen beatmet wurden (eingesetzt wurde Venticute®, eine Lungensurfactant-Zubereitung enthaltend DPPC (Diphosphatidylcholin), POPG (Palmitoyloleylphosphatidylglycerol), Lusupultide, Palmitinsäure und Calciumchlorid). Dieser Befund zeigte sich bereits bei Werten des Fi02 ab 0,5. In Fig. 1 ist zur Veranschaulichung beispielhaft eine Graphik angefügt, die Patienten mit akutem Atemnotsyndrom (ARDS) aufgrund von direkten Lungenschädigungen (Lungenentzündung, Aspiration von Mageninhalt) mit extrem hohen FiO2-Werten zu Behandlungsbeginn (FiO2 über 0,8) darstellt. Die inspiratorische Sauerstofffraktion (Fi02) konnte in der Gruppe der mit der Lungensurfactant-Zubereitung behandelten Patienten wesentlich schneller als in der Gruppe der unbehandelten reduziert werden auf weniger toxische oder nicht mehr toxische inspiratorische Sauerstoff-Fraktionen (oder Sauerstoff-Konzentrationen). Auch die Mortalität als Goldstandard in der Intensivmedizin war an Tag 28 wesentlich geringer in der Gruppe der behandelten (24,1%) als in der Kontrollgrupe (37,5%) (Fig.1). Eine Verringerung der Mortalität zeigte sich ebenso bei Patienten ab Fi02-Werten zu Behandlungsbeginn von 0,5 oder mehr.

### Beschreibung der Figuren

Fig. 1 zeigt einen Vergleich der Überlebensrate in zwei Patientgruppen mit akutem Atemnotsyndrom, die künstlich mit toxischen Sauerstoffkonzentration beatmet wurden, wobei einer der Patientengruppen eine Lungensurfactant-Zubereitung (Venticute®) verabreicht wurde.

## Patentansprüche

1. Verwendung einer Lungensurfactant-Zubereitung zur Herstellung von Arzneimitteln zur Prophylaxe oder Behandlung von akutem Atemnotsyndrom (ARDS) aufgrund von Lungenentzündung oder Aspiration von Mageninhalt.

2. Verwendung nach Anspruch 1, **dadurch gekennzeichnet, dass** man als Lungensurfactant-Zubereitung Venticute^{®} verwendet.

3. Verwendung nach Anspruch 1, **dadurch gekennzeichnet, dass** die verwendete Lungensurfactant-Zubereitung Lusulputide enthält.

4. Verwendung nach Anspruch 1 oder 3, **dadurch gekennzeichnet, dass** die verwendete Lungensurfactant-Zubereitung Bestandteile umfasst, ausgewählt aus (i) DPPC. (ii) POPG, (iii) Palmitinsäure und (iv) Calciumchlorid.

5. Verwendung nach einem der Ansprüche 1 bis 4, **dadurch gekennzeichnet, dass** eine im Vergleich zu beatmeten Patienten, denen keine Lungensurfactant Zubereitung verabreicht wurde, schnellere Absenkung der Konzentration des Sauerstoffs im inspiratorischen Beatmungsgas auf weniger toxische oder nicht mehr toxische Konzentrationen erfolgt.

## Claims

1. Use of a pulmonary surfactant preparation in the manufacture of medicaments for prophylaxis or treatment of acute respiratory distress syndrome (ARDS) on account of pneumonia or aspiration of gastric contents.

2. Use according to Claim 1, **characterized in that** the pulmonary surfactant preparation used is Venticute^{®}.

3. Use according to Claim 1, **characterized in that** the pulmonary surfactant preparation used contains lusulputide.

4. Use according to Claim 1 or 3, **characterized in that** the pulmonary surfactant preparation used comprises constituents selected from (i) DPPC, (ii) POPG, (iii) palmitic acid and (iv) calcium chloride.

5. Use according to any one of Claims 1 to 4, **characterized in that** in comparison with respirated patients to whom no pulmonary surfactant preparation has been administered, a faster lowering of the oxygen concentration in the inspiratory respiration gas to concentrations which are less toxic or no longer toxic is effected.

## Revendications

1. Utilisation d'une préparation de surfactant pulmonaire pour la fabrication de médicaments destinés à la prophylaxie ou au traitement du syndrome aigu de détresse respiratoire de l'adulte (SDRA) dû à une inflammation pulmonaire ou à une aspiration du contenu gastrique.

2. Utilisation selon la revendication 1, **caractérisée en ce qu'**on utilise comme préparation de surfactant pulmonaire du Venticute®.

3. Utilisation selon la revendication 1, **caractérisée en ce que** la préparation de surfactant pulmonaire utilisée contient du lusulputide.

4. Utilisation selon la revendication 1 ou 3, **caractérisée en ce que** la préparation de surfactant pulmonaire comprend des composants choisis parmi (i) la DPPC, (ii) le POPG, (iii) l'acide palmitique et (iv) le chlorure de calcium.

5. Utilisation selon l'une quelconque des revendications 1 à 4, **caractérisée en ce que** par comparaison avec des patients sous respiration artificielle, auxquels n'a été administrée aucune préparation de surfactant pulmonaire, il se produit un abaissement plus rapide de la concentration de l'oxygène dans le gaz inspiratoire de respiration artificielle, jusqu'à des concentrations.moins toxiques ou qui ne sont plus toxiques.
